# EUROPÄISCHE PATENTSCHRIFT

(11) **EP 4 236 710 B1**
(45) Veröffentlichungstag und Bekanntmachung des Hinweises auf die Patenterteilung: **08.04.2026**
(21) Anmeldenummer: 21801110.4
(22) Anmeldetag: 26.10.2021
(51) Int. Cl.: A24F 40/46, A24F 40/44, A24F 40/10, A24F 40/70

(54) **VERDAMPFERVORRICHTUNG FÜR EINEN INHALATOR UND VERFAHREN ZUR HERSTELLUNG EINES INHALATORS**
EVAPORATOR DEVICE FOR AN INHALER, AND METHOD FOR PRODUCING AN INHALER
DISPOSITIF DE VAPORISATION CONÇU POUR UN INHALATEUR ET PROCÉDÉ DE PRODUCTION D'UN INHALATEUR

(30) Priorität: 27.10.2020 DE 102020128184
(43) Veröffentlichungstag der Anmeldung: 06.09.2023
(73) Patentinhaber: Körber Technologies GmbH, 21033 Hamburg (DE)
(72) Erfinder: SCHNUR, Johan, 87448 Waltenhofen (DE); HUESGEN, Till, 87545 Burgberg (DE)
(74) Vertreter: Müller Verweyen
(86) Internationale Anmeldenummer: PCT/EP2021/079671
(87) Internationale Veröffentlichungsnummer: WO 2022/090220

(56) Entgegenhaltungen:
- EP-B1- 2 850 956
- WO-A1-2019/211311
- US-A1- 2019 183 180

## Beschreibung

Die vorliegende Erfindung betrifft eine Verdampfervorrichtung. Des Weiteren betrifft die Erfindung ein Verfahren zur Herstellung einer Verdampfervorrichtung. Es ist hinlänglich bekannt, in elektronischen Zigaretten Verdampferköpfe auf Basis des Docht-Wendel-Prinzips einzusetzen. Neben den weit verbreiteten Docht-Wendel-E-Zigarette existieren als weitere Ansätze die flächige Verdampfung von Liquid direkt an der Liquid-Oberfläche; dieses Prinzip ist beispielsweise aus der Patentanmeldung DE 10 2016 120 803 bekannt.

Ferner ist es aus der DE 10 2017 130 501 A1 bekannt, einen sogenannten Folienverdampfer einzusetzen. Hierbei ist ein Träger aus einem Schichtsystem mit einer Polymerfolie und mindestens einer die Polymerfolie flächig kontaktierende Metallfolie vorgesehen. Die Polymerfolie weist wenigstens eine fluiddurchlässige erste Öffnung auf und die Metallfolie wenigstens eine mit der ersten Öffnung kommunizierende fluiddurchlässige zweite Öffnung, wobei die Metallfolie die Heizelemente ausbildet, und wobei die Heizelemente die zweite Öffnung begrenzend angeordnet sind.

Ferner ist auch aus der WO 2016/166670 A1 ein Folienverdampfer bekannt, der eine flexible Folie mit einem elektrisch leitenden Netzwerk mit Widerstandszonen, die Heizmittel definieren, aufweist. Ferner ist ein flexibler Film vorgesehen, auf dem Abschnitte mit aromatischer Substanz vorgesehen sind, die gegenüber den Heizmitteln angeordnet sind, so dass diese Substanz verflüchtigt werden kann. Ein ganz ähnliches Prinzip ist auch aus der EP 3 282 872 B1 bekannt.

Aus der EP 3 099 190 B1 ist eine sinusförmiges Verdampferfolie bekannt mit einem Materialflächenstück, das zum Erwärmen und Aufsaugen einer Lösung ausgelegt ist, und eine Riffelung umfasst.

Die WO 2019/211311 A1 offenbart eine Heizanordnung zum Bilden eines Aerosols. Mittels eines Kapillarkörpers wird eine Flüssigkeit von einem Flüssigkeitsspeicherabschnitt zu einer elektrischen Heizung befördert. Die elektrische Heizung ist auf einer Außenfläche des Kapillarkörpers angeordnet und umfasst eine Heizschicht, die eine poröse Folie aus elektrisch leitfähigem Material umfasst.

Bei den aus dem Stand der Technik bekannten Lösungen tritt häufig das Problem auf, dass Flüssigkeit nicht in ausreichender Menge verdampft werden kann. Da die Verdampfungsmenge jedoch im Wesentlichen von der Oberflächengröße der Verdampferfolie abhängig ist, geht die Steigerung der Verdampfungsmenge bei den aus dem Stand der Technik bekannten Lösungen zwangsläufig auch mit einem größeren Platzbedarf einher. Gerade die Kompaktheit ist jedoch ein wesentliches Qualitätsmerkmal elektronischer Zigarettenprodukte oder medizinischer Inhalatoren.

Die Aufgabe der Erfindung ist es, eine verbesserte Verdampfervorrichtung für einen Inhalator, einen verbesserten Inhalator sowie eine verbesserte Kartusche bereitzustellen. Ferner ist es Aufgabe der Erfindung, ein verbessertes Verfahren zum Betrieb und zur Herstellung einer Verdampfervorrichtung anzugeben.

Die Erfindung löst die Aufgabe mit den Merkmalen der unabhängigen Ansprüche. Weitere bevorzugte Ausführungsformen der Erfindung sind den Unteransprüchen, den Figuren und der dazugehörigen Beschreibung zu entnehmen.

Ein medizinischer Inhalator im Sinne der Erfindung ist eine Vorrichtung, die geeignet ist, Medikamente oder homöopathische Mittel inhalierbar zu machen bzw. zur Inhalation durch einen Patienten bereit zu stellen. Beispiele für durch einen solchen medizinischen Inhalator verdampfbare Substanzen sind z.B. Cannabinoide, bspw. Cannabidiol oder Tetrahydrocannabinol, Schmerzmittel oder im Zusammenhang mit der Behandlung von Asthma, COPD oder anderen Lungenleiden oder anderen Krankheiten oder Beschwerden wirksame Mittel.

Ein elektronisches Zigarettenprodukt im Sinne der Erfindung ist eine Vorrichtung, die geeignet ist, Flüssigkeiten oder Flüssigkeitsgemische, z.B. umfassend Polyglykol, Glyzerin und optional Nikotin und optional Aroma- und/oder Geschmacksstoffe inhalierbar zu machen bzw. zur Inhalation durch einen Nutzer bereit zu stellen.

Erfindungsgemäß wird vorgeschlagen, dass die Verdampferfolie wenigstens einen Versorgungskanal bildet, über den die wenigstens eine erste Aufnahmeöffnung mit Flüssigkeit von der Dochtstruktur versorgbar ist.

Unter einem Versorgungskanal im Sinne dieser Anmeldung ist ein Kanal zur Leitung einer Flüssigkeit zu verstehen, wobei die Flüssigkeit innerhalb des Kanals durch wenigstens zwei gegenüberliegende Flächen des Kanals geleitet wird.

Der Versorgungskanal ermöglicht es, die erste Aufnahmeöffnung der Verdampferfolie räumlich von der Dochtstruktur zu trennen, so dass die Verdampferfolie in den Abschnitten, in denen die Verdampferfolie den wenigstens einen Versorgungskanal bildet, thermisch von der Dochtstruktur und dem Flüssigkeitsspeicher entkoppelt werden kann. Die Erfindung hat erkannt, dass auf diese Weise der energetische Wirkungsgrad der Verdampfervorrichtung deutlich verbessert werden kann, weil die durch die Metallfolie, die als Heizelement ausgebildet ist, eingebrachte Energie zielgerichtet zur Verdampfung der Flüssigkeit eingesetzt werden kann. Eine unerwünschte Übertragung der Wärmeenergie über die Dochtstruktur auf den Flüssigkeitsspeicher bzw. auf die in dem Flüssigkeitsspeicher enthaltene Flüssigkeit kann damit reduziert werden. Dadurch kann eine Verdampfervorrichtung bei gleicher Batterieladung länger ohne erneuten Ladevorgang betrieben werden.

Ferner kann durch den wenigstens einen Versorgungskanal die zur Verdampfung nutzbare Oberfläche der Verdampferfolie ohne Vergrößerung der Grundfläche, also ohne erhöhten Platzbedarf, vergrößert werden. So kann die Verdampfungsmenge pro Inhalationszug gesteigert werden, ohne dass zusätzliche Dochtmaterialien benötigt werden.

Es hat sich als vorteilhaft erwiesen als Dochtstruktur ein Vlies, beispielsweise ein Glasfaservlies zu verwenden.

Es hat sich ferner als vorteilhaft erwiesen, die ohnehin vorhandene Verdampferfolie selbst zur Bildung des Versorgungskanals zu verwenden. Die Verdampferfolie liegt über die Grundfläche an der Dochtstruktur an, so dass die von der Dochtstruktur an die Verdampferfolie abzugebende Flüssigkeit in effizienter und zuverlässiger Art und Weise in den Versorgungskanal eingeleitet werden kann. Bei der Grundfläche im Sinne dieser Anmeldung handelt es sich um diejenige Fläche, über die die Verdampferfolie mit der Dochtstruktur in Kontakt steht.

Der Versorgungskanal kann beispielsweise dazu eingerichtet sein, mehrere der ersten Aufnahmeöffnungen der Verdampferfolie mit Flüssigkeit zu versorgen.

Eine erste Aufnahmeöffnung kann beispielsweise mit genau einer oder mehreren Abgabeöffnungen in strömungstechnischer Verbindung stehen. Genauso ist es beispielsweise auch möglich, dass mehrere erste Aufnahmeöffnung mit genau einer oder mehreren Abgabeöffnungen in strömungstechnischer Verbindung stehen.

Durch die Bildung des wenigstens einen Versorgungskanals mittels der Verdampferfolie kann die ohnehin vorhandene Polymerfolie verwendet werden, um den Versorgungskanal zu bilden. Somit wird auch bei Bildung des wenigstens einen Versorgungskanals die Metallfolie von der Flüssigkeit im Versorgungskanal geschützt.

Vorzugsweise ist die Verdampferfolie derart ausgeführt, dass die Metallfolie vollständig in die Polymerfolie eingebettet ist. In die Polymerfolie ist die Metallfolie, die vorzugsweise eine mäanderförmige Struktur aufweist, derart eingebettet, dass sich innerhalb der Polymerfolie Abschnitte mit und ohne Metallfolie abwechseln. Die Durchgangsöffnungen sind dann in den Bereichen vorgesehen, in denen keine Metallfolie eingebettet ist. Durch diese Ausgestaltung der Verdampferfolie kann sichergestellt werden, dass auch während des Betriebs das Liquid nicht mit der Metallfolie in Kontakt treten kann. Es können damit die Systemzuverlässigkeitseigenschaften, insbesondere die Lebensdauer und die Ausfallsicherheit, der Verdampfervorrichtung verbessert werden. Ferner kann dadurch ausgeschlossen werden, dass die Metallfolie und das Fluid chemisch zu einem gesundheitsschädlichen Stoff reagieren.

Bei den nachfolgend dargestellten Ausführungsformen sind auch beliebige Kombinationen zweier oder mehrerer Ausführungsformen möglich.

Gemäß einem weiteren Ausführungsbeispiel ist der wenigstens eine Versorgungskanal durch eine Falte der Verdampferfolie gebildet. Durch das Falten der Verdampferfolie lässt sich mit einfachen fertigungstechnischen Maßnahmen ein Versorgungskanal herstellen. Die Verdampferfolie ist damit kostengünstig herstellbar.

Weiterhin ist es vorteilhaft, wenn die Falte zwei sich gegenüberliegende Faltflächen umfasst, die über wenigstens eine Faltkante miteinander verbunden sind, wobei wenigstens 20 %, vorzugsweise wenigstens 30 %, weiter vorzugsweise wenigstens 50 %, der gegenüberliegenden Faltflächen parallel zueinander ausgerichtet sind. Durch die wenigstens teilweise parallele Anordnung der beiden Faltflächen kann eine geeignete Dimensionierung des Versorgungskanals gewährleistet werden. Ferner kann dadurch die Kapillarwirkung bestmöglich vordefiniert werden.

Beispielsweise beträgt die Erstreckung der Falte gesehen entlang einer Richtung orthogonal zu einer benachbarten Grundfläche der Verdampferfolie wenigstens dem 5-fachen der Höhe der Verdampferfolie, weiter beispielsweise dem 10-fachen, insbesondere beispielsweise dem 20-fachen. Die so erreichte räumliche Ausgestaltung der Verdampferfolie schafft bei gleichbleibender Grundfläche eine vergrößerte Oberfläche, die zur Verdampfung der Flüssigkeit genutzt werden kann. Die Höhe der Verdampferfolie beträgt vorzugsweise zwischen 25 µm und 50 µm beträgt, weiter vorzugsweise zwischen 30 µm und 40 µm.

Die Falte ist vorzugsweise derart ausgerichtet, dass sie in Bezug auf die benachbarte Grundfläche der Verdampferfolie bzw. der Oberseite des Trägerelements einen Winkel zwischen 30° und 150° einschließt, weiter vorzugsweise zwischen 80° und 100° und insbesondere einen Winkel von 90°.

Da die ersten Aufnahmeöffnungen vorzugsweise an den Faltflächen vorgesehen sind, wird die fluiddurchlässige Durchgangsöffnung in einer Richtung parallel zu der Grundfläche durchströmt, wenn die Falte orthogonal zu der Grundfläche ausgerichtet ist.

Des Weiteren wird vorgeschlagen, dass mehrere der Falten vorgesehen sind, wobei die Falten durch einen Zwischenabschnitt der Verdampferfolie voneinander beabstandet sind. Durch die Mehrzahl an Falten kann der mit dem Versorgungskanal bzw. der Falte einhergehende positive Effekt noch weiter gesteigert werden. Der Zwischenabschnitt zwischen zwei Falten bewirkt, dass die Falten ausreichend weit voneinander beabstandet sind, sodass die verdampfte Flüssigkeit ungehindert aus den an den Falten vorgesehenen Abgabeöffnungen ausströmen kann. Die Verdampfervorrichtung kann somit energetisch effizienter ausgestaltet und die Verdampfungsmenge gesteigert werden. Vorzugsweise sind die Faltkanten der mehreren Falten parallel zueinander angeordnet.

Gemäß einer weiteren Ausführungsform ist wenigstens eine zweite Aufnahmeöffnung vorgesehen, die an der Grundfläche angeordnet ist, wobei die wenigstens eine zweite Aufnahmeöffnung unmittelbar über die Dochtstruktur mit Flüssigkeit versorgbar ist. Die zweite Aufnahmeöffnung ist ergänzend zu der wenigstens einen ersten Aufnahmeöffnungen vorgesehen, so dass die Menge an erzeugbarem Dampf gesteigert werden kann. Vorzugsweise sind mehrere zweite Aufnahmeöffnungen an der Grundfläche vorgesehen. Durch die Kombination der wenigstes einen zweiten Aufnahmeöffnung, die der Grundfläche zugeordnet ist, und der wenigstens einen ersten Aufnahmeöffnung, die über den wenigsten einen Versorgungskanal mit Flüssigkeit versorgt wird, kann die Menge an verdampfter Flüssigkeit und gleichzeitig die energetische Effizienz der Verdampfervorrichtung gesteigert werden. Vorzugsweise sind die zweiten Aufnahmeöffnungen und die mit ihnen strömungstechnisch verbundenen Abgabeöffnungen beidseitig in Bezug auf die Falte angeordnet, so dass eine Verdampfung von Flüssigkeit beidseitig der Falte sowohl an der Oberseite der Verdampferfolie als auch an den Außenflächen der Falte erfolgen kann.

Der vorangehend genannte Zwischenabschnitt zwischen zwei benachbarten Falten ist insbesondere dann vorteilhaft, wenn der Zwischenabschnitt wenigstens eine der zweiten Aufnahmeöffnungen umfasst. Der Zwischenabschnitt umfasst nämlich vorzugsweise auch die Grundfläche, so dass zwischen zwei Falten zusätzlich noch die Abgabe von verdampfter Flüssigkeit über eine oder mehrere Abgabeöffnungen möglich ist, die über eine oder mehrere der zweiten Aufnahmeöffnungen mit Flüssigkeit versorgt werden. Durch die an dem Zwischenabschnitt vorgesehenen Abgabeöffnungen kann die Verdampfungsmenge noch weiter gesteigert werden.

Es wird weiter vorgeschlagen, dass wenigstens 20 %, beispielsweise wenigstens 30 %, weiter beispielsweise wenigstens 50 % der flächigen Erstreckung der Verdampferfolie zur Bildung des Versorgungskanals dienen.

Unter einer flächigen Erstreckung der Verdampferfolie im Sinne dieser Anmeldung ist die Fläche zu verstehen, die die Verdampferfolie im ausgebreiteten Zustand maximal einnehmen kann, also ohne das Vorhandensein von Falten, Biegungen oder sonstige Umformungen. Üblicherweise befindet sich die Verdampferfolie vor dem Einbau in die Verdampfervorrichtung in solche einem maximal ausgebreiteten Zustand.

Durch das vorgeschlagene Verhältnis zwischen der flächigen Erstreckung der Verdampferfolie und dem flächigen Anteil zur Ausbildung des wenigstens einen Versorgungskanals kann sichergestellt werden, dass der mit den Versorgungskanälen einhergehende technische Effekt, nämlich die thermische Isolation, besonders vorteilhaft zu tragen kommt und gleichzeitig eine ausreichende Menge an zu verdampfender Flüssigkeit über die Versorgungskanäle der Verdampferfolie zugeführt werden kann.

Üblicherweise wird in den Bereichen der Verdampferfolie, in denen sie den Versorgungskanal ausbildet, kein Kontakt mit der Dochtstruktur möglich sein. Deshalb wird weiter vorgeschlagen, dass beispielsweise wenigsten 10 % der flächigen Erstreckung der Verdampferfolie die Grundfläche bildet, weiter beispielsweise wenigstens 20 %, insbesondere beispielsweise wenigstens 30 %. Das Anliegen der Grundfläche an der Dochtstruktur ist vorteilhaft, um die Flüssigkeit effizient in den wenigstens einen Versorgungskanal zu leiten, denn die Grundfläche geht direkt in den Versorgungskanal über. Weiterhin bietet eine entsprechende Mindestgröße der Grundfläche den Vorteil, dass - sofern vorhanden - die wenigstens eine zweite Aufnahmeöffnung, die an der Grundfläche vorgesehen sein kann, ausreichend mit Flüssigkeit versorgt werden kann.

Gemäß einer weiteren Ausführungsform wird vorgeschlagen, dass der wenigstens eine Versorgungskanal dazu eingerichtet ist, die Flüssigkeit von der Dochtstruktur entlang einer Hauptströmungsrichtung zu der wenigstens einen ersten Aufnahmeöffnung zu leiten, wobei die Hauptströmungsrichtung mit der benachbarten Grundfläche einen Winkel zwischen 30° und 150°, insbesondere einen Winkel zwischen 80° und 100°, einschließt. Durch diese Ausrichtung des Strömungskanals kann die wenigstens eine erste Aufnahmeöffnung in einer anderen Ebene als die Grundfläche angeordnet werden. Dadurch wird neben der thermischen Entkoppelung auch die Möglichkeit geschaffen, die Geometrie der Verdampferfolie flexibler zu gestalten. Die Verdampferfolie kann somit nämlich eine räumliche Geometrie aufweisen, die über die übliche flächige Erstreckung von Folienstrukturen hinausgeht. So kann die zum Verdampfen von Flüssigkeit nutzbare Fläche vergrößert werden, ohne dass die Grundfläche vergrößert werden muss.

Wenn die Grundfläche als plane Ebene ausgeführt ist, dann ergibt sich die Bestimmung des Winkels zwischen der Grundfläche und der Hauptströmungsrichtung von selbst. Sofern die Grundfläche gekrümmt ist, wird als Bezugsfläche die Fläche gewählt, die unmittelbar an den Versorgungskanal angrenzt. Sofern mehrere gekrümmte Flächen an den Versorgungskanal angrenzen, wird der Winkel zwischen der Hauptströmungsrichtung und einer fiktiven Grundfläche, die durch Mittelung der Ausrichtung der angrenzenden Grundflächen bestimmt wird, herangezogen.

Es wird weiter vorgeschlagen, dass eine Erstreckung des wenigstens einen Versorgungskanals in Orthogonalrichtung in Bezug auf die benachbarte Grundfläche wenigstens dem 5-fachen, weiter vorzugsweise dem 10-fachen, der Höhe der Verdampferfolie entspricht. Es hat sich gezeigt, dass diese Erstreckung des Versorgungskanals in Orthogonalrichtung einen idealen Kompromiss zwischen einerseits einer ausreichenden thermischen Isolation und andererseits einer zufriedenstellenden Versorgung der wenigsten einen ersten Aufnahmeöffnung mit zu verdampfender Flüssigkeit bietet. Damit kann insgesamt die energetische Effizienz der Verdampfervorrichtung weiter verbessert werden.

Die der Falte zuzuordnenden Durchgangsöffnungen weisen vorzugsweise in Orthogonalrichtung in Bezug auf die Oberseite der Verdampferfolie, die der Grundfläche gegenüber liegt, einen Abstand zu der Oberseite auf, der mehr als 50 % der Erstreckung der Falte in orthogonaler Richtung in Bezug auf die Grundfläche entspricht, weiter vorzugsweise mehr als 60 %, insbesondere vorzugsweise mehr als 70 %. Somit wird in dem Versorgungskanal eine ausreichend großes Flüssigkeitssäule gebildet, die eine thermische Entkoppelung der als Heizelement ausgeführten Metallfolie von der Dochtstruktur und dem Flüssigkeitsspeicher ermöglicht.

Es wird weiter vorgeschlagen, dass der wenigstens eine Versorgungskanal derart dimensioniert ist, dass die Flüssigkeit von der Dochtstruktur zu der wenigsten einen ersten Aufnahmeöffnung unter der Wirkung von Kapillarkräften erfolgt. Durch die Ausnutzung der Kapillarkräfte kann eine zuverlässigere Versorgung des wenigstens einen Versorgungskanals mit zu verdampfender Flüssigkeit sichergestellt werden.

Erfindungsgemäß ist ein Trägerelement vorgesehen, an dessen Oberseite die Verdampferfolie angeordnet ist, und an dessen Unterseite der Flüssigkeitsspeicher angeordnet ist, wobei eine Öffnung zur Aufnahme der Dochtstruktur vorgesehen ist, wobei die Öffnung die Oberseite strömungstechnisch mit der Unterseite verbindet. Vorzugsweise ist die Verdampferfolie, bzw. eine darin umfasste Metallfolie, welche als Heizelement betreibbar ist, über ein Verbindungsmittel in Form einer Löt- oder Sinterverbindung an der Oberseite des Trägerelements befestigt. Es können beispielsweise auch mehrere Flüssigkeitsspeicher, beispielsweise genau zwei Flüssigkeitsspeicher, vorgesehen sein, die mit einer gemeinsamen Dochtstruktur verbunden sind. Die Flüssigkeitsspeicher sind vorzugsweise an der Unterseite des Trägerelements angeordnet. Selbstverständlich kann jedem Flüssigkeitsspeicher auch eine eigene Dochtstruktur zugeordnet sein. Damit ist eine homogene Versorgung der Dochtstruktur mit Flüssigkeit gewährleistet. Es kann beispielsweise auch ein einziger Flüssigkeitsspeicher vorgesehen sein, der einen Steg aufweist, so dass die Dochtstruktur zwischen dem Flüssigkeitsspeicher und der Verdampferfolie eingespannt ist; dadurch kann die Flüssigkeitsversorgung der Verdampferfolie über die Dochtstruktur verbessert werden. Die entsprechende Anordnung der Komponenten gegenüber dem Trägerelement ermöglicht eine stabile Konstruktion, die auch Erschütterungen standhalten kann, wobei gleichzeitig eine zuverlässige Versorgung der Verdampferfolie mit zu verdampfender Flüssigkeit über die in die Öffnung eingesetzte Dochtstruktur ermöglicht wird.

Gemäß einem weiteren Ausführungsbeispiel wird vorgeschlagen, dass die Grundfläche eine ebene Fläche ist. Alternativ ist die Grundfläche die Teilmantelfläche eines Zylinders. Durch eine solche Ausgestaltung der Grundfläche, die ja mit der Dochtstruktur in Kontakt steht, kann eine vorteilhafte Benetzung der Verdampferfolie mit Flüssigkeit erfolgen, so dass der wenigstens eine an die Grundfläche angrenzende Versorgungskanal ausreichend mit Flüssigkeit versorgt werden kann.

Wenn die Grundfläche in Form einer Mantelfläche eines Zylinders ausgestaltet ist, ist es vorteilhaft, wenn die Dochtstruktur eine zylindrische Form mit einer Längsachse aufweist, wobei die Dochtstruktur von der Grundfläche der Verdampferfolie umgeben ist, wobei sich eine Hauptströmungsrichtung des wenigstens einen Versorgungskanals in Bezug auf die Längsachse der Dochtstruktur in Radialrichtung erstreckt. Die der Dochtstruktur gegenüberliegende Fläche der Verdampferfolie korrespondiert so mit der Geometrie der Dochtstruktur, sodass die Verdampferfolie ausreichend mit zu verdampfender Flüssigkeit benetzt werden kann.

Im Falle einer Dochtstruktur mit zylindrischer Grundform wird weiter vorgeschlagen, dass die Verdampferfolie von einem Außenring umgeben ist, so dass sich zwischen der Verdampferfolie und dem Außenring ein Strömungskanal ausbildet. Der Außenring und die Dochtstruktur weisen vorzugsweise eine gemeinsame Längsachse auf. Der Außenring weist dann zur Bildung des Strömungskanals einen Innenradius auf, der wenigstens der maximalen radialen Erstreckung der Verdampferfolie entspricht. Durch den so ausgebildeten Strömungskanal, können die Abgabeöffnungen besonders vorteilhaft umströmt werden, sodass die Verdampfungsmenge weiter gesteigert werden kann.

Die eingangs genannte Aufgabe wird ebenfalls gelöst durch einen Inhalator und eine Kartusche umfassend eine Verdampfervorrichtung nach einem der Ansprüche 1 bis 13.

Des Weiteren wird die eingangs genannte Aufgabe gelöst durch ein Verfahren zum Betreiben einer Verdampfervorrichtung nach einem der Ansprüche 1 bis 13, wobei zur Erzeugung des Flüssigkeitsdampfes die Verdampferfolie mit einer elektrischen Heizspannung beaufschlagt wird.

Schließlich wird die eingangs genannte Aufgabe gelöst durch ein Verfahren zur Herstellung einer Verdampfervorrichtung, wobei die Verdampfervorrichtung eine auf einem Trägerelement angeordnete Verdampferfolie umfasst, wobei die Verdampferfolie wenigstens einen Versorgungskanal bildet, mit dem die Verdampferfolie über eine Dockstruktur mit zu verdampfender Flüssigkeit aus einem Flüssigkeitsspeicher versorgbar ist, wobei der Versorgungskanal durch eine Falte der Verdampferfolie gebildet ist, wobei das Verfahren die folgenden Schritte umfasst:
a) Bereitstellen eines Basismaterials für die Herstellung der Verdampferfolie umfassend ein Substrat aus Polyimid mit einer einseitig darauf aufgebrachten Kupferkaschierung;
b) Strukturierung der Kupferkaschierung durch ein Ätzverfahren, so dass eine Kupferstruktur auf dem Substrat gebildet wird;
c) Aufbringen einer Polyimid-Deckschicht auf die Seite des Substrats mit der Kupferstruktur;
d) Erzeugen einer Durchgangsöffnung, über die eine Oberseite der Verdampferfolie strömungstechnisch mit einer Unterseite der Verdampferfolie verbindbar ist, mittels eines Lasers;
e) Anschneiden einer Faltkante mittels eines Lasers, so dass ein Einschnitt entsteht;
f) Falten der Verdampferfolie entlang der eingeschnittenen Faltkante;
g) Verbinden der gefalteten Verdampferfolie mit dem Trägerelement.

Durch das vorgeschlagene Verfahren kann auf einfache Weise eine Verdampferfolie mit einer Faltung hergestellt werden, wobei durch die Falte der Versorgungskanal gebildet wird. Durch das Herstellungsverfahren kann zum einen eine sehr dünne Verdampferfolie hergestellt werden, zum anderen kann auch eine Faltung mit sehr engem Biegeradius durch die angeschnittene Faltkante realisiert werden. Dadurch kann die Oberfläche der Verdampferfolie, über die die verdampfte Flüssigkeit abgegeben werden kann, gesteigert werden, so dass insgesamt das Verdampfungsverhalten durch das Herstellungsverfahren verbessert werden kann. Als Polyimid für das Substrat kann beispielsweise Pyralux^{®} HT oder Pyralux ^{®} AP der Firma DuPont verwendet werden. Die Schichtdicke des Substrats beträgt vorzugsweise zwischen 5 und 50 µm auf, weiter beispielsweise zwischen 20 und 30 µm und insbesondere vorzugsweise 25 µm. Die Schichtdicke der Kupferkaschierung beträgt vorzugsweise zwischen 1 und 10 µm, weiter vorzugsweise zwischen 2 und 8 µm, insbesondere vorzugsweise 5 µm. Die so hergestellte Verdampferfolie bietet den Vorteil, dass durch die Falte ein Versorgungskanal gebildet wird, über den die Durchgangsöffnungen der Verdampferfolie dann mit zu verdampfender Flüssigkeit versorgt werden kann.

Es wird weiter vorgeschlagen, dass in dem Verfahrensschritt b) die folgenden Teilschritte vorgenommen werden:
b₁) Aufbringen eines Fotolacks auf die Kupferkaschierung;
b₂) Belichten des Fotolacks in vordefinierten Bereichen;
b₃) Entwickeln des Fotolacks, so dass der verbleibende Fotolack eine Schutzschicht für die Kupferkaschierung in den vordefinierten Bereichen bildet;
b₄) Aufbringen eines Ätzmittels auf die Seite mit dem entwickelten Fotolack, so dass die Kupferkaschierung nur in den vordefinierten Bereichen auf dem Substrat verbleibt;
b₅) Entfernen des restlichen Fotolacks.

Der Fotolack, auch Fotoresist genannt, kann beispielsweise auflaminiert werden. Das anschließende Belichten kann beispielsweise mittels einer Direktbeleuchtung erfolgen, bei der gezielt nur die vordefinierten Bereiche angeleuchtet werden, oder beispielsweise mittels einer Maskenbelichtung. Bei der Maskenbelichtung wird eine Maske auf den Fotolack aufgelegt, die abschnittsweise lichtdurchlässig ausgeführt ist, so dass die zu belichtenden Bereiche von einer Lichtquelle gezielt belichtet werden können. Nach dem Entwickeln des Fotolacks bleiben nur die vordefinierten Bereiche des Fotolacks auf der Kupferkaschierung bestehen, so dass die Kupferkaschierung an diesen Stellen geschützt ist. Durch das anschließende Aufbringen einer ätzenden Substanz auf die Seite mit der Kupferkaschierung und dem entwickelten Fotolack, wird das Kupfer an den Stellen, an denen der entwickelte Fotolack nicht seine schützende Wirkung entfaltet, weggeätzt. Die Kupferkaschierung verbleibt damit nur noch an vordefinierten Stellen auf dem Substrat, so dass auf dem Substrat eine Kupferstruktur entsteht. In einem anschließenden Schritt wird der Fotolack wieder entfernt, man spricht vom sogenannten Strippen, so dass nur noch das Substrat mit der Kupferstruktur verbleibt. Von der vollflächigen Kupferkaschierung verbleibt also eine Kupferstruktur.

Vorzugsweise erfolgt in dem Verfahrensschritt c) das Aufbringen einer Polyimid-Deckschicht durch Auflaminieren in einer Vakuumpresse. Durch die so aufgebrachte Polyimid-Deckschicht kann eine Vorspannung des Substrats mit der darauf aufgebrachten Kupferstruktur erfolgen und zwar derart, dass das Substrat auf der Seite mit der Polyimid-Deckschicht eine konkave Biegung aufweist. Die konkave Biegung hat sich als vorteilhaft erwiesen, weil so eine Falte mit einer besonders spitz zulaufenden Faltkante ermöglicht wird. Damit trotz der Krümmung eine einfache Weiterverarbeitung möglich ist, wird das Substrat mit der Kupferstruktur und Polyimid-Deckschicht zur weiteren Verarbeitung vorzugsweise auf einer planen Oberfläche eines Trägers im vorgespannten Zustand fixiert. Als Material für die Polyimid-Deckschicht kann beispielsweise Pyralux ^{®} HT0100 der Firma DuPont verwendet werden.

Es wird weiter vorgeschlagen, dass in dem Verfahrensschritt e) zusätzlich mittels des Lasers Kontaktierungsstellen für die Versorgung der Verdampferfolie mit einer elektrischen Heizspannung freigelegt werden. Unter dem Freilegen der Kontaktierungsstellen ist das Abtragen der Polyimid-Deckschicht an den entsprechenden Stellen gemeint. Auf diese Weise wird eine besonders kostengünstige Möglichkeit geschaffen, die Verdampferfolie mit der entsprechenden Heizspannung zu versorgen.

Es wird ferner vorgeschlagen, dass in dem Verfahrensschritt e) zusätzlich die Verdampferfolie für die Verbindung mit dem Trägerelement zugeschnitten wird. Dies ermöglicht es beispielsweise, ein großflächiges Schichtsystem zu erzeugen, aus dem eine Mehrzahl an Verdampferfolien ausgeschnitten werden kann. So kann auf kostengünstige Weise die Verdampferfolie in großen Stückzahlen hergestellt werden. Durch das Zuschneiden kann dann aus dem großflächigen Schichtsystem eine Verdampferfolie gebildet werden, deren Abmessungen derart sind, dass sie mit einem Trägerelement der Verdampfervorrichtung verbunden werden kann. Dies geschieht vorzugsweise derart, dass der durch die Falte gebildete Versorgungskanal unmittelbar die Dochtstruktur kontaktiert, die in einer Öffnung des Trägerelements eingesetzt ist.

Die Erfindung wird im Folgenden anhand bevorzugter Ausführungsformen unter Bezugnahme auf die beigefügten Figuren erläutert. Dabei zeigt
- Fig. 1: eine schematische Schnittansicht einer Verdampfervorrichtung mit einem Versorgungskanal;
- Fig. 2: eine schematische Schnittdarstellung einer Verdampferfolie;
- Fig. 3: eine schematische Schnittansicht einer Verdampfervorrichtung mit zwei Versorgungskanälen;
- Fig. 4: eine Schnittansicht einer Verdampfervorrichtung mit einer zylinderförmigen Dochtstruktur;
- Fig. 5: eine schematische Schnittdarstellung einer Falte einer Verdampferfolie;
- Fig. 6: die Schritte a) und b) eines Herstellungsverfahrens einer Verdampfervorrichtung;
- Fig. 7: die Schritte c) bis e) eines Herstellungsverfahrens einer Verdampfervorrichtung;
- Fig. 8: eine Verdampferfolie nach dem Durchführen des Verfahrensschrittes f) aus einer ersten Perspektive;
- Fig. 9: eine Verdampferfolie nach dem Durchführen des Verfahrensschrittes f) aus einer zweiten Perspektive;
- Fig. 10: eine Verdampfervorrichtung nach dem Durchführen des Verfahrensschrittes g) aus einer perspektivischen Ansicht.

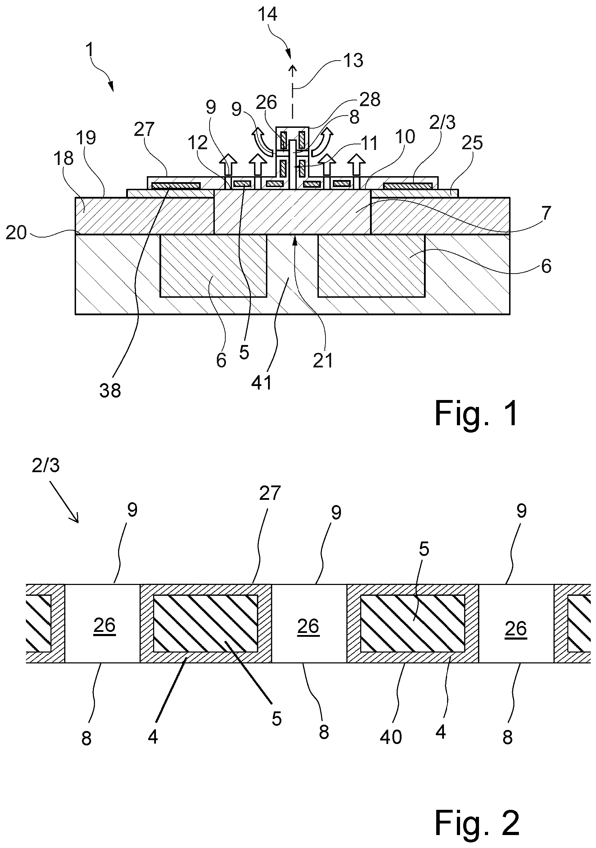

Figur 1 zeigt eine Verdampfervorrichtung 1 umfassend eine Verdampferfolie 2, die über ein Verbindungsmittel 25 an einer Oberseite 19 eines Trägerelements 18 befestigt ist, einen Flüssigkeitsspeicher 6, der an einer Unterseite 20 des Trägerelements 18 angeordnet sind, und eine Dochtstruktur 7, die dazu eingerichtet ist, die Verdampferfolie 2 mit zu verdampfender Flüssigkeit aus dem Flüssigkeitsspeicher 6 zu versorgen.

Selbstverständlich ist es grundsätzlich auch möglich, zwei oder mehrere Flüssigkeitsspeicher 6 vorzusehen.

Das Trägerelement 18 weist eine Öffnung 21 auf, in die die Dochtstruktur 7 eingesetzt ist. So kann die zu verdampfende Flüssigkeit aus dem Flüssigkeitsspeicher 6 mittels der Dochtstruktur 7 durch die Öffnung 21 bis zur Verdampferfolie 2 gefördert werden. Ferner weist der Flüssigkeitsspeicher 6 einen Steg 41 auf, über den die Dochtstruktur 7 zwischen dem Flüssigkeitsspeicher 6 und der Verdampferfolie 2 eingepresst werden kann. Als Dochtstruktur 7 ist bei den dargestellten Ausführungsformen ein Vlies, beispielsweise ein Glasfaservlies vorgesehen.

Die Verdampferfolie 2, bzw. eine darin umfasste Metallfolie 5, welche als Heizelement betreibbar ist, ist vorzugsweise über ein Verbindungsmittel 25 in Form einer Löt- oder Sinterverbindung mit dem Trägerelement 18 verbunden.

Eine beispielhafte Darstellung der Verdampferfolie 2 ist in Figur 2 schematisch dargestellt. Die Verdampferfolie 2 umfasst ein Schichtsystem 3, das wiederum eine Polymerfolie 4 und die wenigstens eine die Polymerfolie 4 flächig kontaktierende Metallfolie 5 umfasst. Vorzugsweise ist die Polymerfolie 4 auf die Metallfolie 5 auflaminiert. In dem Ausführungsbeispiel gemäß Figur 2 ist die Metallfolie 5 ganzseitig von der Polymerfolie 4 umgeben. Die Metallfolie 5 ist somit vollständig in die Polymerfolie 4 eingepackt. Es kann somit sichergestellt werden, dass auch während des Betriebs das Liquid nicht mit der Metallfolie in Kontakt treten kann. Bei der Polymerfolie 4 handelt es sich vorzugsweise um eine Polyimidfolie.

Die Verdampferfolie 2 ist perforiert, so dass eine erste Aufnahmeöffnung 8 an einer Unterseite 40 strömungstechnisch mit einer Abgabeöffnung 9 an einer Oberseite 27 verbunden werden kann. Die erste Aufnahmeöffnung 8 und die Abgabeöffnung 9 bilden so zusammen jeweils eine fluiddurchlässige Durchgangsöffnung 26.

Die in Figur 1 dargestellte Dochtstruktur 7 besteht vorzugsweise aus einem porösen und/oder kapillaren Material, das aufgrund von Kapillarkräften in der Lage ist, von der Verdampferfolie 2 verdampfte Flüssigkeit in ausreichender Menge von dem Flüssigkeitsspeicher 6 zu der Verdampferfolie 2 passiv nachzufördern, um ein Leerlaufen der Durchgangsöffnung 26 und sich daraus ergebende Probleme zu verhindern.

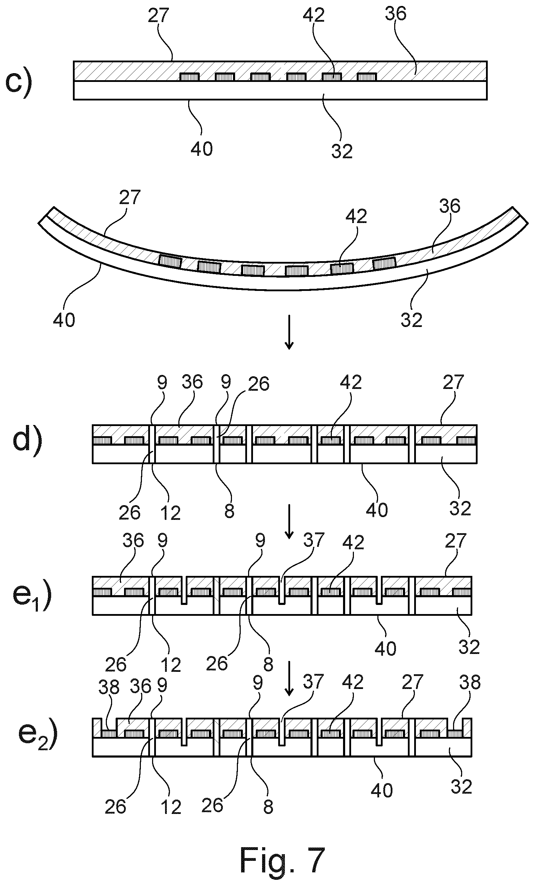

Die Metallfolie 5 gemäß Figur 2 ist als Heizelement ausgeführt, beispielsweise in Form eines ohmschen Heizers. Bei Verwendung der Verdampferfolie 2 aus Figur 2 als Bestandteil der Verdampfervorrichtung 1 aus Figur 1 kommt es zur Verdampfung der Flüssigkeit innerhalb der Durchgangsöffnungen 26, wobei die Flüssigkeit beispielsweise durch Kapillarkräfte über die Dochtstruktur 7 aus dem Flüssigkeitsspeicher 6 nachgefördert wird. Im Betriebszustand kommt es in bestimmten Phasen, beispielsweise wenn ein Inhalationszug durch einen Nutzer detektiert wird, zu einer Aktivierung der Metallfolie 5, durch Anliegen einer Heizspannung an zwei Kontaktierungsstellen 38 (siehe Figuren 7 bis 10) der Metallfolie 5 und Flie-ßen eines elektrischen Stroms durch die Metallfolie 5, so dass diese sich erwärmt. Es kommt infolgedessen auch zu einer Wärmeerzeugung in der fluiddurchlässigen Durchgangsöffnung 26. Beim Durchströmen der fluiddurchlässige Durchgangsöffnung 26 ausgehend von der ersten Aufnahmeöffnung 8 wird die Flüssigkeit so stark erhitzt, dass sie den dampfförmigen Aggregatzustand erreicht und als Dampf die Abgabeöffnungen 9 verlässt. Somit wird innerhalb der Durchgangsöffnung 26 in Folge der Verdampfung Volumen frei, in das aufgrund der Kapillarkräfte Flüssigkeit nachkriechen kann. Die weitere Versorgung mit Flüssigkeit erfolgt über den Versorgungskanal 11 und die Dochtstruktur 7 aus dem Flüssigkeitstank 6. Während des Verdampfungsvorgangs in der Durchgangsöffnung 26 kommt es somit kontinuierlich zu einer Nachförderung von Flüssigkeit aus den Flüssigkeitsspeichern 6 über die Dochtstruktur 7.

Wie aus Figur 1 ersichtlich ist, werden die beiden ersten Aufnahmeöffnungen 8 nicht unmittelbar über die Dochtstruktur 7 mit Flüssigkeit versorgt. Die Versorgung erfolgt über einen strömungstechnisch zwischen die erste Aufnahmeöffnung 8 und die Dochtstruktur 7 geschalteten Versorgungskanal 11, der durch die Verdampferfolie 2 selbst gebildet ist.

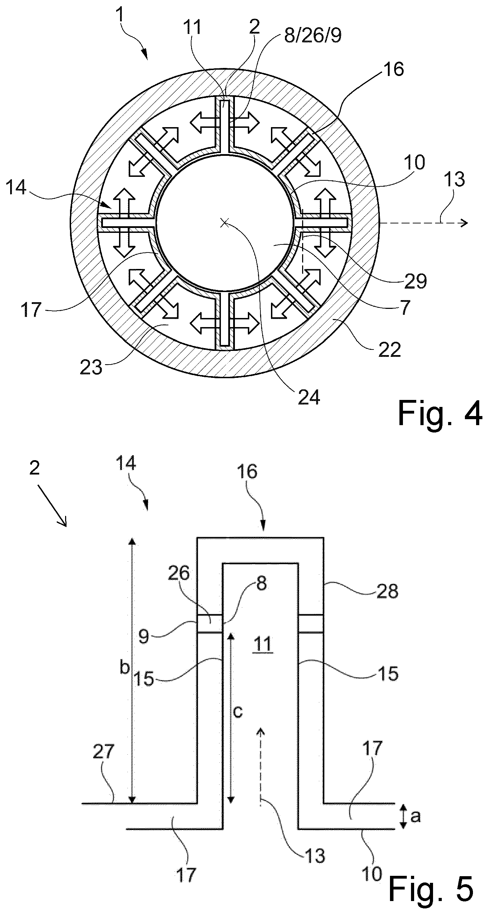

Der Versorgungskanal 11 ist durch eine Falte 14 der Verdampferfolie 2 gebildet, die in Figur 5 vergrößert in einer Schnittansicht dargestellt ist. Durch die Falte 14 der Verdampferfolie 2 werden zwei sich gegenüberliegende Faltflächen 15 gebildet, die über eine Faltkante 16 miteinander verbunden sind. Die einander gegenüberliegenden Faltflächen 15 einer Falte 14 können unregelmäßig geformt sein, sind aber beispielsweise in Teilabschnitten oder Teilflächen deren Anteil an der Gesamtfläche der Faltflächen 15 beispielsweise mehr als 20 % betragen parallel zueinander ausgerichtet. Eine parallele Erstreckung der sich gegenüberliegenden Faltflächen 15 ist vorteilhaft, weil so die Wirkung der Kapillarkräfte bestmöglich vordefiniert werden kann.

Vorzugsweise beträgt die Erstreckung b der Falte 14 in einer Richtung orthogonal zu einer benachbarten Grundfläche 10 der Verdampferfolie 2 wenigstens dem 5-fachen der Höhe a der Verdampferfolie 2. Daraus ergibt sich eine räumlich ausgeprägtere Gestaltung der Verdampferfolie 2 im Vergleich zu den sonst nur flächig verlegten Verdampferfolien 2 aus dem Stand der Technik.

Die Falte 14 ist derart ausgestaltet, dass diese senkrecht in Bezug auf die Grundfläche 10 bzw. auf eine Oberseite 19 des Trägerelements 18 ausgerichtet ist, vgl. Figur 1. Mit einer senkrechten Ausrichtung der Falte 14 ist gemeint, dass deren Faltflächen 15 orthogonal zu der benachbarten Grundfläche 10 ausgerichtet sind.

Damit ist auch der durch die Falte 14 gebildete Versorgungskanal 11 senkrecht in Bezug auf die Grundfläche 10 ausgerichtet. Aus dieser Gestaltung der Falte 14 resultiert eine Hauptströmungsrichtung 13 der Flüssigkeit innerhalb des Versorgungskanals 11. Die Hauptströmungsrichtung 13 ist diejenige Strömungsrichtung, in die der Großteil der Flüssigkeit ausgehend von der Dochtstruktur 7 bis zu einer ersten Aufnahmeöffnung 8 innerhalb des wenigstens einen Versorgungskanals 11 strömt. Die Hauptströmungsrichtung 13 ist dementsprechend auch orthogonal in Bezug auf die der Falte 14 benachbarte Grundfläche 10 ausgerichtet.

Die beiden ersten Aufnahmeöffnungen 8 sind an den Faltflächen 15 vorgesehen, so dass die fluiddurchlässigen Durchgangsöffnungen 26 in einer Richtung durchströmt werden, die parallel zu der Grundfläche 10, ausgerichtet ist.

Ferner weist die Falte 14 in Figur 5 genau zwei der fluiddurchlässigen Durchgangsöffnungen 26 auf. Selbstverständlich kann die Falte 14 auch nur eine einzige der Durchgangsöffnungen 26 aufweisen oder mehr als zwei der Durchgangsöffnungen 26. Sofern mehrere Durchgangsöffnungen 26 vorgesehen sind, können diese auch hintereinander in Bezug auf eine Achse senkrecht zur Zeichenebene der Figur 5 angeordnet sein. Alternativ oder zusätzlich können die Durchgangsöffnungen 26 auch übereinander in Bezug auf eine Achse orthogonal zu der Grundfläche 10 angeordnet sein.

Die in der Figur 5 dargestellten Durchgangsöffnungen 26 weisen in Orthogonalrichtung in Bezug auf die Oberseite 27 der Verdampferfolie 2, die der Grundfläche 10 gegenüber liegt, einen Abstand c zu der Oberseite 27 auf, der mehr als 50 % der Erstreckung b in orthogonaler Richtung der Falte 14 entspricht, vorzugsweise mehr als 60 %, weiter vorzugsweise mehr als 70 %. Durch diese Beabstandung der Durchgangsöffnungen 26 und damit auch der ersten Aufnahmeöffnungen 14 von der Oberseite 27 kann eine ausreichende Flüssigkeitssäule in dem Versorgungskanal 11 sichergestellt werden, so dass eine thermische Entkoppelung der als Heizelement ausgeführten Metallfolie 5 ermöglicht wird. Die sich in der Falte 14 befindliche Flüssigkeitssäule bewirkt die Isolation der im Betrieb Wärmeenergie abgebenden Metallfolie 5 gegenüber der Dochtstruktur 7 und den beiden Flüssigkeitsspeichern 6, so dass die energetische Effizienz der Verdampfervorrichtung 1 gesteigert werden kann.

Beim Betreiben der Verdampfervorrichtung 1 aus Figur 1 wird durch die Wärmeentwicklung in der fluiddurchlässigen Durchgangsöffnung 26 die zu verdampfende Flüssigkeit von den Flüssigkeitsspeichern 6 mittels der Dochtstruktur 7 und dem Versorgungskanal 11 in die Durchgangsöffnung 26 gefördert. Dort wird die Flüssigkeit mindestens bis zur Siedetemperatur erhitzt, so dass das Fluid verdampft und im gasförmigen Zustand aus der Abgabeöffnung 9 entweicht. Während dieses Vorgangs, also während die Metallfolie 5 aktiviert ist, d.h. heizt, wird kontinuierlich Flüssigkeit aus den Flüssigkeitsspeichern 6 mittels der Dochtstruktur 7 und dem bzw. durch den Versorgungskanal 11 nachgefördert, sodass eine kontinuierliche Verdampfung von Flüssigkeit erfolgen kann.

Ferner ist der Figur 1 zu entnehmen, dass die Abgabe des Dampfes nicht nur über die Abgabeöffnungen 9, die einer Außenfläche 28 senkrecht zur Grundfläche 10 der Falte 14 zugeordnet sind, erfolgt. Es sind zusätzlich an der Verdampferfolie 2 auch Abgabeöffnungen 9 an der Oberseite 27 der Verdampferfolie 2, die der Grundfläche 10 gegenüber liegt, angeordnet. Diese Abgabeöffnungen 9 sind strömungstechnisch mit zweiten Aufnahmeöffnungen 12 verbunden, die unmittelbar über die Dochtstruktur 7 mit Flüssigkeit versorgbar sind. Die zweiten Aufnahmeöffnungen 12 werden also nicht über den Versorgungskanal 11 mit Flüssigkeit versorgt. Die Durchgangsöffnungen 26, die den zweiten Aufnahmeöffnungen 12 zugeordnet sind, können aber genauso ausgeführt sein, wie diejenigen, die über den Versorgungskanal 11 mit Flüssigkeit versorgt werden; es sei diesbezüglich auf die Ausführungen zu Figur 2 verwiesen.

Durch die Kombination von ersten Aufnahmeöffnungen 8, die über den Versorgungskanal 11 versorgt werden, und zweiten Aufnahmeöffnungen 12, die direkt über die Dochtstruktur 7 versorgt werden, kann eine Abgabe von verdampfter Flüssigkeit in unterschiedliche Raumrichtungen erfolgen, sodass insgesamt die Oberfläche der Verdampferfolie 2, die für die Verdampfung über die Abgabeöffnungen 9 genutzt werden kann, vergrößert wird. Somit kann insgesamt die Verdampfungsmenge pro Inhalationszug gesteigert werden.

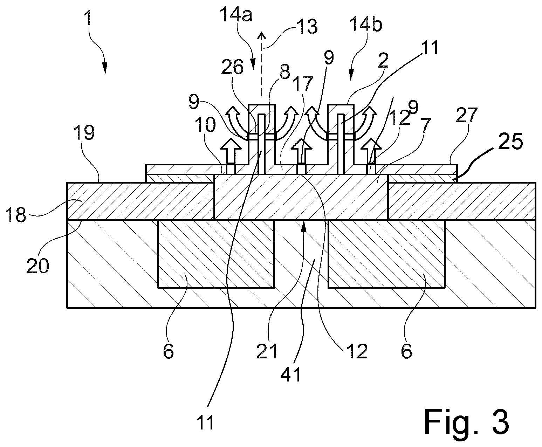

Figur 3 zeigt eine Verdampfervorrichtung 1 mit demselben Grundaufbau wie die Verdampfervorrichtung 1 aus Figur 1, allerdings mit dem Unterschied, dass neben der ersten Falte 14a eine zweite Falte 14b vorgesehen ist, die von der ersten Falte 14a durch einen Zwischenabschnitt 17 beabstandet ist.

Jede der beiden Falten 14a und 14b weist jeweils zwei erste Aufnahmeöffnungen 8 auf sowie diesen zugeordnete Abgabeöffnungen 9. Selbstverständlich kann die Anzahl der Aufnahmeöffnungen 8 und die damit korrelierende Anzahl an Durchgangsöffnungen 26 wie bereits bei dem Ausführungsbeispiel gemäß Figur 1 erläutert wurde, beliebig verändert werden.

Ferner umfasst auch der Zwischenabschnitt 17 wenigstens eine zweite Aufnahmeöffnung 12, die unmittelbar über die Dochtstruktur 7 mit Flüssigkeit versorgt wird. Zusätzlich ist links der ersten Falte 14a und rechts der zweiten Falte 14b jeweils eine weitere zweite Aufnahmeöffnung 12 vorgesehen. Diese Anordnung hat sich als vorteilhaft erwiesen, weil jede der Falten 14a und 14b beidseitig ausgehend von den zweiten Aufnahmeöffnung 12 mit verdampfter Flüssigkeit umströmt werden kann und gleichzeitig ausgehend von den ersten Aufnahmeöffnungen 8, die den Falten 14a und 14b selbst zugeordnet sind, eine zusätzliche Umströmung der Falten 14a und 14b ermöglicht wird. So kann die Verdampfungsmenge pro Inhalationszug deutlich gesteigert werden und gleichzeitig durch die Isolationswirkung der in den Versorgungskanälen 11 der ersten und zweite Falte 14a und 14b vorhandenen Flüssigkeitssäule die thermische Entkopplung der Metallfolie 5 gegenüber der Dochtstruktur 7 bzw. den beiden Flüssigkeitsspeichern 6 gesteigert werden.

Selbstverständlich können auch mehr als zwei Falten 14a und 14b vorgesehen sein, wodurch der Effekt noch weiter gesteigert werden kann. Sofern mehrere Falten 14a und 14b vorgesehen sind, sind diese vorzugsweise parallel zueinander ausgerichtet, d.h. die Faltkanten 16 der mehreren Falten 14a und 14b sind parallel zueinander ausgerichtet. Ferner sind beispielsweise sämtliche Hauptströmungsrichtungen 13 in den durch die Falten 14 gebildeten Versorgungskanälen 11 orthogonal zu den an die jeweilige Falte 14 angrenzende Grundfläche 10 ausgerichtet.

Figur 4 zeigt eine Ausführungsform der Verdampfervorrichtung 1 mit einer zylinderförmigen Dochtstruktur 7 mit einer Längsachse 24 der Dochtstruktur 7, die senkrecht zur Zeichenebene ausgerichtet ist. Es ist zu erkennen, dass die Verdampferfolie 2 die Mantelfläche der zylindrischen Dochtstruktur 7 umgibt, wobei eine Vielzahl von Falten 14 vorgesehen ist, die sich in Bezug auf die Längsachse 24 in Radialrichtung erstrecken. Damit ist gemeint, dass die Faltkante 16, vgl. auch Figur 5, in Radialrichtung in Bezug auf die Längsachse 24 am weitesten von der Längsachse 24 entfernt ist. Die Faltkante 16 ist dann beispielsweise parallel zu der Längsachse 24 ausgerichtet.

Bei dem Ausführungsbeispiel gemäß Figur 4 werden durch eine Vielzahl von Falten 14 die Versorgungskanäle 11 gebildet. Die Verdampfung der Flüssigkeit erfolgt bei diesem Ausführungsbeispiel ausschließlich über Durchgangsöffnungen 26, die über erste Aufnahmeöffnungen 8 über einen Versorgungskanal 11 mit Flüssigkeit versorgt werden.

Selbstverständlich können ergänzend zu den Durchgangsöffnungen 26, die den Falten 14 zugeordnet sind, auch Durchgangsöffnungen 26 vorgesehen sein, die unmittelbar über die Dochtstruktur 7, also über eine zweite Aufnahmeöffnung 12 mit Flüssigkeit versorgt werden. So kann beispielsweise zwischen jeder Falte 14 wenigsten eine Durchgangsöffnung 26 vorgesehen sein, die über eine zweite Aufnahmeöffnung 12 mit Flüssigkeit versorgt wird.

Bei einer zylinderförmigen Dochtstruktur 7 ist die Grundfläche 10 nicht eben, sondern eine gekrümmte Fläche. Wenn in diesem Fall die Hauptströmungsrichtung 13 in dem Versorgungskanal 2 orthogonal zu der benachbarten Grundfläche 10 ausgerichtet ist, dann bezieht sich das bei der in Figur 4 dargestellten Schnittdarstellung auf eine Tangente 29 durch den Punkt der Grundfläche 10, der unmittelbar an die Falte 14 angrenzt.

Der entsprechende Flüssigkeitsspeicher 6, der die Dochtstruktur 7 mit Flüssigkeit versorgt, ist in der Figur 4 nicht dargestellt. Dieser kann aber beispielsweise in Axialrichtung in Bezug auf die Längsachse 24 hinter und/oder vor der Dochtstruktur 7 angeordnet sein. Dementsprechend wird die zu verdampfende Flüssigkeit in Axialrichtung von der Dochtstruktur 7 aus dem Flüssigkeitsspeicher 6 befördert und dann in Radialrichtung in Bezug auf die Längsachse 24 umgelenkt, um das zu verdampfende Flüssigkeit an die Verdampferfolie 2 bzw. an den durch die Verdampferfolie 2 gebildeten Versorgungskanal 11 abzugeben.

Die Verdampferfolie 2 ist in dem Ausführungsbeispiel gemäß Figur 4 von einem Außenring 22 umgeben, so dass sich zwischen der Verdampferfolie 2 und dem Außenring 22 eine Mehrzahl von Strömungskanälen 23 ausbildet. Bei einem Inhalationszug durch einen Benutzer werden die Vielzahl der Strömungskanäle 23 dann in Richtung der Längsachse 24 durchströmt.

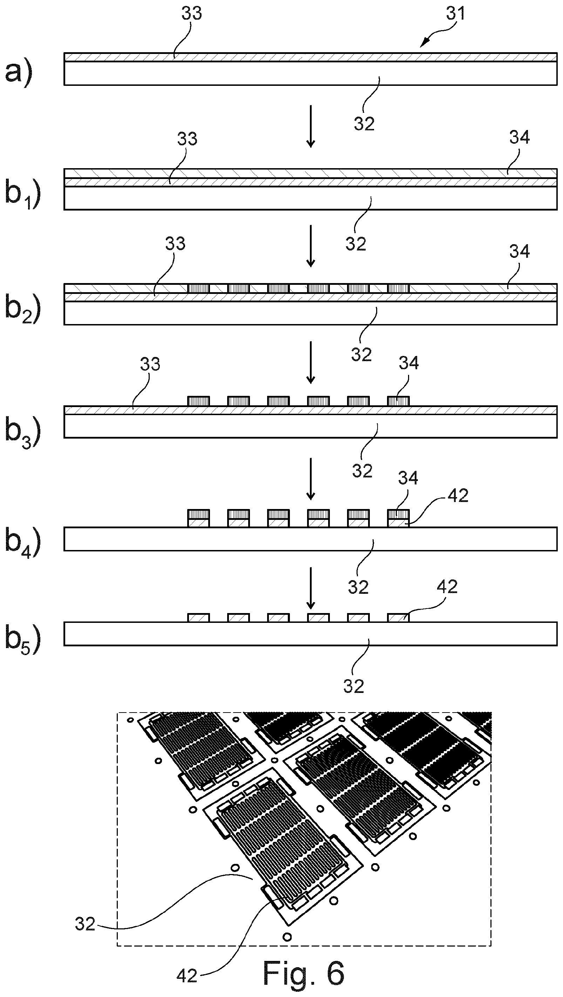

Die Figur 6 zeigt die Verfahrensschritte a) und b) eines Verfahrens 30 zur Herstellung der Verdampfervorrichtung 1. In einem ersten Verfahrensschritt a) wird ein Basismaterial 31 umfassend ein Substrat 32 und ein eine darauf aufgebrachte Kupferkaschierung 33 bereitgestellt.

Der darauffolgende Verfahrensschritt b), in dem die Kupferkaschierung 34 strukturiert wird, so dass eine Kupferstruktur 42 entsteht, untergliedert sich in die Verfahrensschritte b₁) bis b₅). Im Verfahrensschritt b₁) wird auf die Kupferkaschierung 33 ein Fotolack 34 auflaminiert, der im Verfahrensschritt b₂) in vordefinierten Bereichen belichtet wird. Dies kann beispielsweise durch entsprechende Direktbeleuchtung oder mittels einer Maske erfolgen. Im Verfahrensschritt b₃) wird der Fotolack 34 entwickelt, d.h. nur eine aus der Belichtung resultierende Lackmaske verbleibt auf der Kupferkaschierung 33; der übrige Fotolack 34 wird entfernt. Im Verfahrensschritt b₄) wird ein Ätzmittel auf die Seite mit dem entwickelten Fotolack 34 aufgebracht, so dass die Kupferkaschierung an den Stellen weggeätzt wird, an denen kein Schutz durch die durch den Fotolack 34 gebildete Lackmaske besteht. Aus der vollflächigen Kupferkaschierung 33 wird somit eine Kupferstruktur 42 gebildet. In Verfahrensschritt b₅) wird dann der übrige Fotolack 34 entfernt, so dass auf dem Substrat 32 nur die Kupferstruktur 42 verbleibt. Vorzugsweise weist die Kupferstruktur 42 eine Mäanderform auf. Das Ergebnis des Verfahrensschrittes b), nämlich die auf dem Substrat 32 aufgebrachte Kupferstruktur 42 ist in Figur 6 unten schematische dargestellt.

Figur 7 zeigt die Verfahrensschritte c) bis e) des Verfahrens 30. Im Verfahrensschritt c) wird auf der Seite des Substrats 32 mit der Kupferstruktur 42 eine Polyimid-Deckschicht 36 aufgebracht, so dass die die Metallfolie 5 (vgl. Figur 2) bildende Kupferstruktur 42 vollständig in Schichten aus Polyimid, also dem aus Polyimid gebildeten Substrat 32 und der Polyimid-Deckschicht 36, eingebettet ist. Die Polyimid-Deckschicht 36 wird einseitig in einer Vakuumpresse auflaminiert, was dazu führt, dass es zu einer Krümmung des so entstehenden Zwischenproduktes kommt und zwar derart, dass die Oberseite 27, also die Seite mit der Polyimid-Deckschicht 36, konkav gekrümmt ist; vgl. Darstellung in Figur 7 c) unten.

Im Verfahrensschritt d) wird das nun vorliegende Zwischenprodukt umfassend das Substrat 32, die Kupferstruktur 42 sowie die Polyimid-Deckschicht 36 dann mittels eines Lasers perforiert, so dass strömungstechnische Verbindungen zwischen der Oberseite 27 und der Unterseite 12 entstehen. Es werden so die Durchgangsöffnungen 26 gebildet, die die ersten und zweiten Aufnahmeöffnungen 8 und 12 jeweils mit den Abgabeöffnungen 9 verbinden; siehe auch Figuren 1 bis 5. Wie in Figur 7 deutlich zu erkennen ist, erfolgt die Perforation ausschließlich in Bereichen, in denen keine Kupferstruktur 42 verläuft, also zwischen zwei Bereichen mit einer Kupferstruktur 42.

Im Verfahrensschritt e₁) wird dann zusätzlich noch eine Faltkante 16 mittels eines Lasers angeschnitten, wobei ein so entstehender Einschnitt 37 vorzugsweise vollständig die Polyimid-Deckschicht 36 durchdringt, das Substrat 32 wird vorzugsweise nur teilweise angeschnitten. Im Verfahrensschritt e₂) werden mittels des Lasers mehrere Kontaktierungsstellen 38 freigelegt, d.h. die Polyimid-Deckschicht 36 wird durch den Laser an der entsprechenden Stelle so weit abgetragen, bis ein Teil der Kupferstruktur 42 freiliegt.

Falls erforderlich kann als Zwischenschritt noch ein Zuschneiden bzw. Zurechtschneiden der Verdampferfolie 2 erfolgen, so dass sie die richtige Größe zum Verbinden mit dem Trägerelement 18 aufweist.

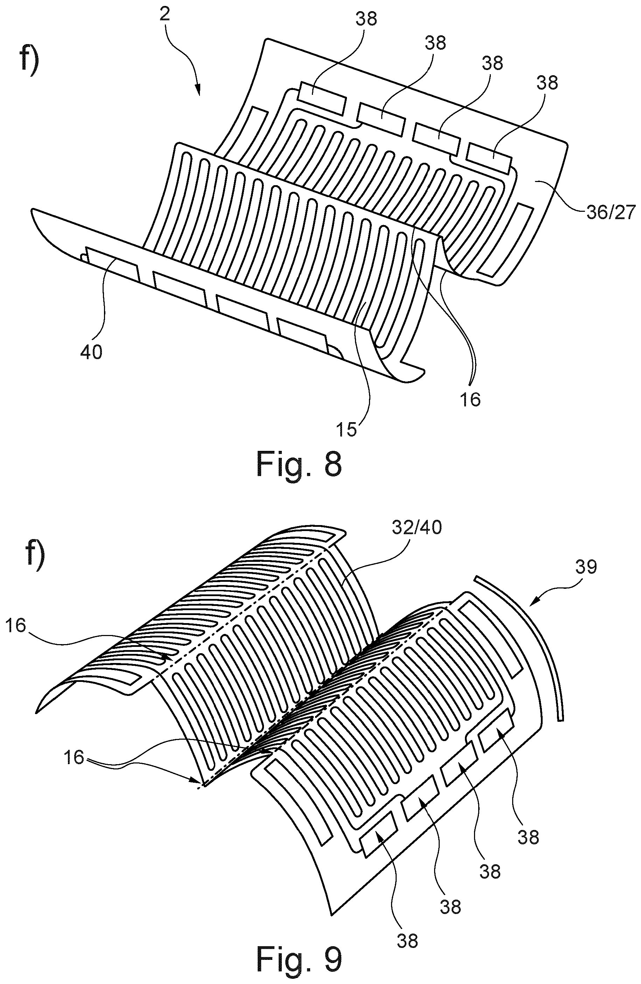

Die Figuren 8 und 9 zeigen die Verdampferfolie 2 in einem gefalteten Zustand, und damit das Ergebnis des Verfahrensschrittes f). Das Falten erfolgt entlang der angeschnittenen Faltkanten 16, so dass scharfe Faltkanten 16 erzielt werden können. Figur 8 zeigt die Verdampferfolie 2 aus einer Perspektive mit Blick auf die Oberseite 27. Es ist deutlich zu erkennen, wir die Faltflächen 15 aufgrund der im Verfahrensschritt c) erzeugten Vorspannung gekrümmt sind. Ferner sind die Kontaktierungsstellen 38 sowie die durch die Polyimid-Deckschicht 36 durschimmernde mäanderförmige Kupferstruktur 42 zu erkennen.

Figur 9 zeigt die Verdampferfolie 2 aus einer Perspektive mit Blick auf die Unterseite 40. Ferner ist eine Krümmung 39 der Verdampferfolie 2 kenntlich gemacht.

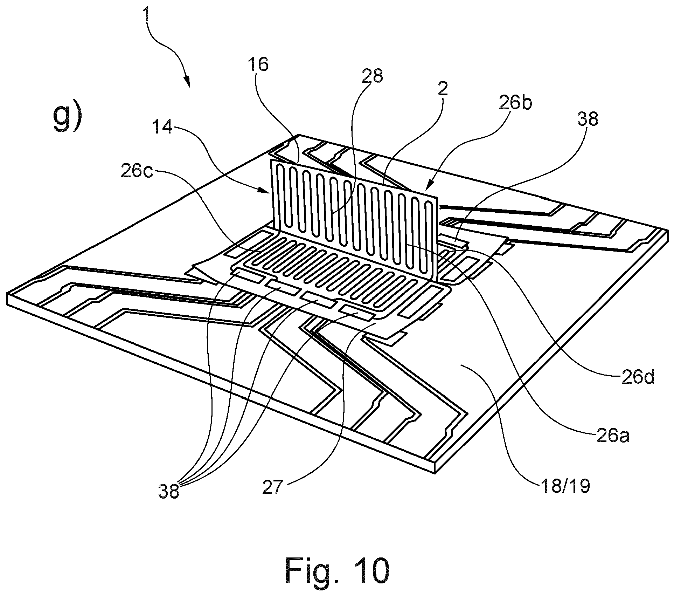

Figur 10 zeigt die Verdampferfolie 2 in einem mit dem Trägerelement 18 verbundenen Zustand, also nach Vollendung des Verfahrensschrittes g).

Figur 10 zeigt eine perspektivische Darstellung einer Verdampfervorrichtung 1 mit ähnlichem Grundaufbau wie die in Figur 1 dargestellte Ausführungsform der Verdampfervorrichtung 1. Es ist zu erkennen, dass die Falte 14 wie eine Finne aus der durch die Oberseite 27 der Verdampferfolie 2 gebildeten Ebene herausragt.

Durch die die im Verfahrensschritt c) erzeugte Krümmung 39 der Verdampferfolie 2 weist die Falte 14 im mit dem Trägerelement 18 verbundenen Zustand eine Vorspannung auf, die dazu führt, dass die Falte 14 eine stabilere Struktur aufweist und die scharfe Faltkante 16 auch dauerhaft, als beispielsweise im Betrieb, in Transportsituationen oder bei unsachgemäßer Handhabung, aufrechterhalten werden kann. Es ist weiterhin zu erkennen, dass die Verdampferfolie 2 mit der Oberseite 19 des Trägerelements 18 verbunden ist. Die Verdampferfolie 2 weist vier Durchgangsöffnungen 26a, 26b (nicht sichtbare Rückseite der Falte 14), 26c und 26d auf, wobei zwei Durchgangsöffnungen 26a und 26b durch jeweils eine erste Aufnahmeöffnung 8 (vgl. Figur 1) und zwei weitere Durchgangsöffnungen 26c und 26d durch jeweils eine zweite Aufnahmeöffnung 12 (vgl. Figur 1) mit zu verdampfender Flüssigkeit versorgt werden. Dementsprechend erfolgt eine Abgabe der verdampften Flüssigkeit beidseitig der Falte 14 sowohl an einer Oberseite 27 der Verdampferfolie 2 und gleichzeitig an den Außenflächen 28 der Falte 14.

Wie vorangehend beschrieben weist die durch die Kupferstruktur 42 gebildete Metallfolie 5 innerhalb der Polymerfolie 4, die aus dem Substrat 32 und dem Polyimid-Deckschicht 36 gebildet ist, mäanderförmige Strukturen auf, wobei die mäanderförmigen Strukturen separat ansteuerbare Abschnitte aufweisen. Separat ansteuerbar bedeutet, dass der entsprechende Abschnitt separate Kontaktierungsstellen 38 aufweist und damit die an dem jeweiligen Abschnitt anliegende Heizspannung individuell einstellbar ist. Hierzu ist der jeweilige Abschnitt von den übrigen Abschnitten der Kupferstruktur 42 elektrisch isoliert. Bei einem separat ansteuerbaren Abschnitt der Kupferstruktur 42 spricht man von einem ansteuerbaren Kanal. Jeder dieser Abschnitte bildet einen ohmschen Heizer, wobei es sich als vorteilhaft erwiesen hat, wenn jeder separat ansteuerbare Abschnitt der Metallfolie 5 bzw. Metallstruktur 42 einen Widerstand zwischen 1 und 3 Ω aufweist, beispielsweise ca. 2 Ω. Vorzugsweise weist das Ausführungsbeispiel in Figur 10 vier separat ansteuerbare Kanäle auf, wobei die Durchgangsöffnungen 26a, 26b, 26c und 26d je einem dieser vier Kanäle zugeordnet sind, so dass die Durchgangsöffnungen 26a bis 26d separat über die Kanäle ansteuerbar sind.

Durch die Befestigung der Verdampferfolie 2 an dem Trägerelement 18 und/oder an der Dochtstruktur 7 mittels des Verbindungsmittels 25 kann die Verdampferfolie 2 zuverlässig in der umgeformten Position gehalten werden.

Mit der Verdampfervorrichtung 1 gemäß Figur 10 ist es beispielsweise möglich, während eines Inhalationszuges von drei Sekunden eine Menge von 7,4 mg Flüssigkeit zu verdampfen; gleichzeitig lässt sich ein energetischer Wirkungsgrad von 60 % und mehr bei üblichen Raumbedingungen erreichen.

## Patentansprüche

1. Verdampfervorrichtung (1) für einen Inhalator, insbesondere für ein elektronisches Zigarettenprodukt oder einen medizinischen Inhalator, umfassend
- eine Verdampferfolie (2),
- einen Flüssigkeitsspeicher (6), und
- eine Dochtstruktur (7), die dazu eingerichtet ist, die Verdampferfolie (2) mit Flüssigkeit aus dem Flüssigkeitsspeicher (6) zu versorgen, wobei
- die Verdampferfolie (2) wenigstens eine erste Aufnahmeöffnung (8) zur Aufnahme von Flüssigkeit und wenigstens eine Abgabeöffnungen (9) zur Abgabe von verdampfter Flüssigkeit aufweist, wobei
- die Verdampferfolie (2) über eine Grundfläche (10) mit der Dochtstruktur (7) in Kontakt steht, wobei
- die Verdampferfolie (2) wenigstens einen Versorgungskanal (11) bildet, über den die wenigstens eine erste Aufnahmeöffnung (8) mit Flüssigkeit von der Dochtstruktur (7) versorgbar ist, **dadurch gekennzeichnet, dass**
- die Verdampferfolie (2) ein Schichtsystem (3) mit einer Polymerfolie (4) und mindestens einer die Polymerfolie (4) flächig kontaktierenden Metallfolie (5), die als Heizelement ausgebildet ist, umfasst, wobei
- ein Trägerelement (18) vorgesehen ist, an dessen Oberseite (19) die Verdampferfolie (2) angeordnet ist, und an dessen Unterseite (20) der Flüssigkeitsspeicher (6) angeordnet ist, wobei
- eine Öffnung (21) zur Aufnahme der Dochtstruktur (7) vorgesehen ist, wobei
- die Öffnung (21) die Oberseite (19) strömungstechnisch mit der Unterseite (20) verbindet.

2. Verdampfervorrichtung (1) nach Anspruch 1, **dadurch gekennzeichnet, dass**
- der wenigstens eine Versorgungskanal (11) durch eine Falte (14) der Verdampferfolie (2) gebildet ist.

3. Verdampfervorrichtung (1) nach Anspruch 2, **dadurch gekennzeichnet, dass**
- die Falte (14) zwei sich gegenüberliegende Faltflächen (15) umfasst, die über wenigstens eine Faltkante (16) miteinander verbunden sind, wobei
- wenigstens 20 % der gegenüberliegenden Faltflächen (15) parallel zueinander ausgerichtet sind.

4. Verdampfervorrichtung (1) nach einem der Ansprüche 2 oder 3, **dadurch gekennzeichnet, dass**
- mehrere der Falten (14) vorgesehen sind, wobei die Falten (14) durch einen Zwischenabschnitt (17) der Verdampferfolie (2) voneinander beabstandet sind.

5. Verdampfervorrichtung (1) nach einem der vorangehenden Ansprüche, **dadurch gekennzeichnet, dass**
- wenigstens eine zweite Aufnahmeöffnung (12) vorgesehen ist, die an der Grundfläche (10) angeordnet ist, wobei
- die wenigstens eine zweite Aufnahmeöffnung (12) unmittelbar über die Dochtstruktur (7) mit Flüssigkeit versorgbar ist.

6. Verdampfervorrichtung (1) nach einem der vorangehenden Ansprüche, **dadurch gekennzeichnet, dass**
- wenigstens 20 % der flächigen Erstreckung der Verdampferfolie (2) den wenigstens einen Versorgungskanal (11) ausbilden.

7. Verdampfervorrichtung (1) nach einem der vorangehenden Ansprüche, **dadurch gekennzeichnet, dass**
- der wenigstens eine Versorgungskanal (11) dazu eingerichtet ist, die Flüssigkeit von der Dochtstruktur (7) entlang einer Hauptströmungsrichtung (13) zu der wenigstens einen ersten Aufnahmeöffnung (8) zu leiten, wobei
- die Hauptströmungsrichtung (13) mit der benachbarten Grundfläche (10) einen Winkel zwischen 30° und 150°, insbesondere einen Winkel zwischen 80° und 100°, einschließt.

8. Verdampfervorrichtung (1) nach einem der vorangehenden Ansprüche, **dadurch gekennzeichnet, dass**
- eine Erstreckung (b) des wenigstens einen Versorgungskanals (11) in Orthogonalrichtung in Bezug auf die benachbarte Grundfläche (10) wenigstens dem 5-fachen der Höhe (a) der Verdampferfolie (2) entspricht.

9. Verdampfervorrichtung (1) nach einem der vorangehenden Ansprüche, **dadurch gekennzeichnet, dass**
- der wenigstens eine Versorgungskanal (11) derart dimensioniert ist, dass die Flüssigkeit von der Dochtstruktur (7) zu der wenigstens einen ersten Aufnahmeöffnung (8) unter der Wirkung von Kapillarkräften erfolgt.

10. Verdampfervorrichtung (1) nach einem der vorangehenden Ansprüche, **dadurch gekennzeichnet, dass**
- die Grundfläche (10) eine ebene Fläche ist.

11. Verdampfervorrichtung (10) nach einem der Ansprüche 1 bis 9, **dadurch gekennzeichnet, dass**
- die Grundfläche (10) die Teilmantelfläche eines Zylinders ist.

12. Verdampfervorrichtung (1) nach Anspruch 11, **dadurch gekennzeichnet, dass**
- die Dochtstruktur (7) eine zylindrische Form mit einer Längsachse (24) aufweist, wobei
- die Dochtstruktur (7) von der Grundfläche (10) der Verdampferfolie (2) umgeben ist, wobei
- sich eine Hauptströmungsrichtung (13) des wenigstens einen Versorgungskanals (11) in Bezug auf die Längsachse (24) der Dochtstruktur (7) in Radialrichtung erstreckt.

13. Verdampfervorrichtung (1) nach Anspruch 12, **dadurch gekennzeichnet, dass**
- die Verdampferfolie (2) von einem Außenring (22) umgeben ist, so dass sich zwischen der Verdampferfolie (2) und dem Außenring (22) ein Strömungskanal (23) ausbildet.

14. Inhalator umfassend eine Verdampfervorrichtung (1) nach einem der vorangehenden Ansprüche.

15. Kartusche für einen Inhalator umfassend eine Verdampfervorrichtung (1) nach einem der Ansprüche 1 bis 13.

16. Verfahren zum Betreiben einer Verdampfervorrichtung (1) nach einem der Ansprüche 1 bis 13, wobei zur Erzeugung des Flüssigkeitsdampfes die Verdampferfolie (1) mit einer elektrischen Heizspannung beaufschlagt wird.

17. Verfahren (30) zur Herstellung einer Verdampfervorrichtung (1), wobei die Verdampfervorrichtung (1) eine auf einem Trägerelement (18) angeordnete Verdampferfolie (2) umfasst, wobei die Verdampferfolie (2) wenigstens einen Versorgungskanal (11) bildet, mit dem die Verdampferfolie (2) über eine Dockstruktur (7) mit zu verdampfender Flüssigkeit aus einem Flüssigkeitsspeicher (6) versorgbar ist, wobei der Versorgungskanal (11) durch eine Falte (14) der Verdampferfolie (2) gebildet ist, wobei das Verfahren (30) die folgenden Schritte umfasst:
a) Bereitstellen eines Basismaterials (31) für die Herstellung der Verdampferfolie (2) umfassend ein Substrat (32) aus Polyimid mit einer einseitig darauf aufgebrachten Kupferkaschierung (33);
b) Strukturierung der Kupferkaschierung (33) durch ein Ätzverfahren, so dass eine Kupferstruktur (42) auf dem Substrat (32) gebildet wird;
c) Aufbringen einer Polyimid-Deckschicht (36) auf die Seite des Substrats (32) mit der Kupferstruktur (42);
d) Erzeugen einer Durchgangsöffnung (26), über die eine Oberseite (27) der Verdampferfolie (2) strömungstechnisch mit einer Unterseite (40) der Verdampferfolie (2) verbindbar ist, mittels eines Lasers;
e) Anschneiden einer Faltkante (16) mittels eines Lasers, so dass ein Einschnitt (37) entsteht;
f) Falten der Verdampferfolie (2) entlang der eingeschnittenen Faltkante (16);
g) Verbinden der gefalteten Verdampferfolie (2) mit dem Trägerelement (18).

18. Verfahren (30) nach Anspruch 17, **dadurch gekennzeichnet, dass** in dem Verfahrensschritt b) die folgenden Teilschritte vorgenommen werden:
b₁) Aufbringen eines Fotolacks (34) auf die Kupferkaschierung (33);
b₂) Belichten des Fotolacks (34) in vordefinierten Bereichen;
b₃) Entwickeln des Fotolacks (34), so dass der verbleibende Fotolack (34) eine Schutzschicht für die Kupferkaschierung (33) in den vordefinierten Bereichen bildet;
b₄) Aufbringen eines Ätzmittels auf die Seite mit dem entwickelten Fotolack (34), so dass die Kupferkaschierung (33) nur in den vordefinierten Bereichen auf dem Substrat (32) verbleibt;
b₅) Entfernen des restlichen Fotolacks (34).

19. Verfahren (30) nach Anspruch 17 oder 18, **dadurch gekennzeichnet, dass** in dem Verfahrensschritt c) das Aufbringen einer Polyimid-Deckschicht (36) durch Auflaminieren in einer Vakuumpresse erfolgt.

20. Verfahren (30) nach einem der Ansprüche 17 bis 19, **dadurch gekennzeichnet, dass** in dem Verfahrensschritt e) zusätzlich mittels des Lasers Kontaktierungsstellen (38) für die Versorgung der Verdampferfolie (2) mit einer elektrischen Heizspannung freigelegt werden.

21. Verfahren (30) nach einem der Ansprüche 17 bis 20, **dadurch gekennzeichnet, dass** in dem Verfahrensschritt e) zusätzlich die Verdampferfolie (2) für die Verbindung mit dem Trägerelement (18) zugeschnitten wird.

## Claims

1. Vaporizer device (1) for an inhaler, in particular for an electronic cigarette product or a medical inhaler, comprising
- a vaporizer film (2),
- a fluid storage reservoir (6), and
- a wick structure (7) which is configured to supply the vaporizer film (2) with liquid from the liquid reservoir (6),
- the vaporizer film (2) having at least one first receiving opening (8) for receiving liquid and at least one discharge opening (9) for discharging vaporized liquid,
- the vaporizer film (2) being in contact with the wick structure (7) via a base surface (10),
- the vaporizer film (2) forming at least one supply channel (11) via which the at least one first receiving opening (8) can be supplied with liquid from the wick structure (7), **characterized in that**
- the vaporizer film (2) comprises a layer system (3) having a polymer film (4) and at least one metal foil (5) which is designed as a heating element and which makes surface contact with the polymer film (4),
- a support element (18) being provided, on the top face (19) of which the vaporizer film (2) is arranged, and on the bottom face (20) of which the liquid reservoir (6) is arranged,
- an opening (21) for receiving the wick structure (7) being provided,
- the opening (21) fluidically connecting the top face (19) to the bottom face (20).

2. Vaporizer device (1) according to claim 1, **characterized in that**
- the at least one supply channel (11) is formed by a fold (14) of the vaporizer film (2).

3. Vaporizer device (1) according to claim 2, **characterized in that**
- the fold (14) comprises two opposing fold surfaces (15) which are connected to each other via at least one fold edge (16),
- at least 20% of the opposing fold surfaces (15) being aligned in parallel with one another.

4. Vaporizer device (1) according to any of claims 2 or 3,
**characterized in that**
- a plurality of the folds (14) are provided, the folds (14) being spaced apart from one another by an intermediate portion (17) of the vaporizer film (2).

5. Vaporizer device (1) according to any of the preceding claims,
**characterized in that**
- at least one second receiving opening (12) is provided which is arranged on the base surface (10),
- it being possible for the at least one second receiving opening (12) to be supplied with liquid directly via the wick structure (7).

6. Vaporizer device (1) according to any of the preceding claims,
**characterized in that**
- at least 20% of the planar extent of the vaporizer film (2) forms at least one supply channel (11).

7. Vaporizer device (1) according to any of the preceding claims,
**characterized in that**
- the at least one supply channel (11) is configured to direct the liquid from the wick structure (7) in a main flow direction (13) to the at least one first receiving opening (8),
- the main flow direction (13) forming, together with the adjacent base surface (10), an angle between 30° and 150°, in particular an angle between 80° and 100°.

8. Vaporizer device (1) according to any of the preceding claims,
**characterized in that**
- an extent (b) of the at least one supply channel (11) in the orthogonal direction with respect to the adjacent base surface (10) corresponds to at least 5 times the height (a) of the vaporizer film (2).

9. Vaporizer device (1) according to any of the preceding claims,
**characterized in that**
- the at least one supply channel (11) is designed such that the liquid is from the wick structure (7) to the at least one first receiving opening (8) under the influence of capillary forces.

10. Vaporizer device (1) according to any of the preceding claims,
**characterized in that**
- the base surface (10) is a flat surface.

11. Vaporizer device (10) according to any of claims 1 to 9,
**characterized in that**
- the base surface (10) is the partial lateral surface area of a cylinder.

12. Vaporizer device (1) according to claim 11, **characterized in that**
- the wick structure (7) has a cylindrical shape with a longitudinal axis (24),
- the wick structure (7) being surrounded by the base surface (10) of the vaporizer film (2),
- a main flow direction (13) of the at least one supply channel (11) extending in a radial direction with respect to the longitudinal axis (24) of the wick structure (7).

13. Vaporizer device (1) according to claim 12, **characterized in that**
- the vaporizer film (2) is surrounded by an outer ring (22) so that a flow channel (23) is formed between the vaporizer film (2) and the outer ring (22).

14. Inhaler comprising a vaporizer device (1) according to any of the preceding claims.

15. Cartridge for an inhaler comprising a vaporizer device (1) according to any of claims 1 to 13.

16. Method for operating a vaporizer device (1) according to any of claims 1 to 13, wherein an electrical heating voltage is applied to the vaporizer film (1) in order to generate the liquid vapor.

17. Method (30) for manufacturing a vaporizer device (1), wherein the vaporizer device (1) comprises a vaporizer film (2) arranged on a support element (18), wherein the vaporizer film (2) forms at least one supply channel (11), by means of which the vaporizer film (2) can be supplied with liquid to be vaporized from a liquid reservoir (6) via a wick structure (7), wherein the supply channel (11) is formed by a fold (14) of the vaporizer film (2), wherein the method (30) comprises the following steps:
a) providing a base material (31) for manufacturing the vaporizer film (2), the base material comprising a substrate (32) made of polyimide having a copper lamination (33) applied to one face thereof;
b) structuring the copper lamination (33) by an etching process so that a copper structure (42) is formed on the substrate (32);
c) applying a polyimide top layer (36) to the face of the substrate (32) that has the copper structure (42);
d) creating a through-opening (26), using a laser, via which through-opening a top face (27) of the vaporizer film (2) can be fluidically connected to a bottom face (40) of the vaporizer film (2);
e) cutting a fold edge (16) using a laser so that a notch (37) is created;
f) folding the vaporizer film (2) along the notched fold edge (16);
g) connecting the folded vaporizer film (2) to the support element (18).

18. Method (30) according to claim 17, **characterized in that** the following sub-steps are carried out in method step b):
b₁) applying a photoresist (34) to the copper lamination (33);
b₂) exposing the photoresist (34) in predefined areas;
b₃) developing the photoresist (34) so that the remaining photoresist (34) forms a protective layer for the copper lamination (33) in the predefined areas;
b₄) applying an etching agent to the face having the developed photoresist (34) so that the copper lamination (33) remains only in the predefined areas on the substrate (32);
b₅) removing the remaining photoresist (34).

19. Method (30) according to claim 17 or 18, **characterized in that,** in method step c), a polyimide top layer (36) is applied by laminating in a vacuum press.

20. Method (30) according to any of claims 17 to 19, **characterized in that,** additionally, in process step e), contact points (38) for supplying the vaporizer film (2) with an electrical heating voltage are exposed by means of the laser.

21. Method (30) according to any of claims 17 to 20, **characterized in that,** additionally, in process step e), the vaporizer film (2) is cut to size for connection to the support element (18).

## Revendications

1. Dispositif de vaporisation (1) pour un inhalateur, en particulier pour un produit du type cigarette électronique ou un inhalateur médical, comprenant :
- une feuille de vaporisation (2),
- un réservoir de liquide (6), et
- une structure de mèche (7) qui est conçue pour alimenter la feuille de vaporisation (2) en liquide provenant du réservoir de liquide (6),
- la feuille de vaporisation (2) présentant au moins une première ouverture de réception (8) pour recevoir du liquide et au moins une ouverture de distribution (9) pour distribuer du liquide vaporisé,
- la feuille de vaporisation (2) est en contact avec la structure de mèche (7) par l'intermédiaire d'une surface de base (10),
- la feuille de vaporisation (2) forme au moins un canal d'alimentation (11) par lequel ladite au moins une première ouverture de réception (8) peut être alimentée en liquide à partir de la structure de mèche (7), **caractérisé en ce que**
- la feuille de vaporisation (2) comprend un système de couches (3) avec une feuille polymère (4) et au moins une feuille métallique (5) en contact plan avec la feuille polymère (4), qui est conçue comme élément chauffant,
- un élément de support (18) est prévu, sur la face supérieure (19) duquel est disposée la feuille de vaporisation (2) et sur la face inférieure (20) duquel est disposé le réservoir de liquide (6),
- une ouverture (21) est prévue pour recevoir la structure de mèche (7),
- l'ouverture (21) relie fluidiquement la face supérieure (19) à la face inférieure (20).

2. Dispositif de vaporisation (1) selon la revendication 1, **caractérisé en ce que**
- ledit au moins un canal d'alimentation (11) est formé par un pli (14) de la feuille de vaporisation (2).

3. Dispositif de vaporisation (1) selon la revendication 2, **caractérisé en ce que**
- le pli (14) comprend deux surfaces de pliage opposées (15) qui sont reliées entre elles par au moins un bord de pliage (16),
- au moins 20 % des surfaces de pliage opposées (15) sont alignées parallèlement les unes aux autres.

4. Dispositif de vaporisation (1) selon l'une des revendications 2 ou 3, **caractérisé en ce que**
- plusieurs plis (14) sont prévus, les plis (14) étant espacés les uns des autres par une partie intermédiaire (17) de la feuille de vaporisation (2).

5. Dispositif de vaporisation (1) selon l'une des revendications précédentes, **caractérisé en ce que**
- au moins une deuxième ouverture de réception (12) est prévue, qui est disposée sur la surface de base (10),
- ladite au moins une deuxième ouverture de réception (12) pouvant être alimentée en liquide directement par la structure de mèche (7).

6. Dispositif de vaporisation (1) selon l'une des revendications précédentes, **caractérisé en ce que**
- au moins 20 % de l'extension plane de la feuille de vaporisation (2) forment ledit au moins un canal d'alimentation (11).

7. Dispositif de vaporisation (1) selon l'une des revendications précédentes, **caractérisé en ce que**
- ledit au moins un canal d'alimentation (11) est conçu pour conduire le liquide de la structure de mèche (7) le long d'une direction d'écoulement principale (13) vers ladite au moins une première ouverture de réception (8),
- la direction d'écoulement principale (13) forme avec la surface de base adjacente (10) un angle compris entre 30° et 150°, en particulier un angle compris entre 80° et 100°.

8. Dispositif de vaporisation (1) selon l'une des revendications précédentes, **caractérisé en ce que**
- une extension (b) dudit au moins un canal d'alimentation (11) dans la direction orthogonale par rapport à la surface de base adjacente (10) correspond à au moins 5 fois la hauteur (a) de la feuille de vaporisation (2).

9. Dispositif de vaporisation (1) selon l'une des revendications précédentes, **caractérisé en ce que**
- ledit au moins un canal d'alimentation (11) est dimensionné de telle sorte que le liquide s'écoule de la structure de mèche (7) vers ladite au moins une première ouverture de réception (8) sous l'effet de forces capillaires.

10. Dispositif de vaporisation (1) selon l'une des revendications précédentes, **caractérisé en ce que**
- la surface de base (10) est une surface plane.

11. Dispositif de vaporisation (10) selon l'une des revendications 1 à 9, **caractérisé en ce que**
- la surface de base (10) est la surface d'enveloppe partielle d'un cylindre.

12. Dispositif de vaporisation (1) selon la revendication 11, **caractérisé en ce que**
- la structure de mèche (7) présente une forme cylindrique avec un axe longitudinal (24),
- la structure de mèche (7) est entourée par la surface de base (10) de la feuille de vaporisation (2),
- une direction d'écoulement principale (13) dudit au moins un canal d'alimentation (11) s'étend dans une direction radiale par rapport à l'axe longitudinal (24) de la structure de mèche (7).

13. Dispositif de vaporisation (1) selon la revendication 12, **caractérisé en ce que**
- la feuille de vaporisation (2) est entourée d'une bague extérieure (22) de sorte qu'un canal d'écoulement (23) se forme entre la feuille de vaporisation (2) et la bague extérieure (22).

14. Inhalateur comprenant un dispositif de vaporisation (1) selon l'une des revendications précédentes.

15. Cartouche pour un inhalateur comprenant un dispositif de vaporisation (1) selon l'une des revendications 1 à 13.

16. Procédé pour faire fonctionner un dispositif de vaporisation (1) selon l'une des revendications 1 à 13, dans lequel, pour produire la vapeur de liquide, la feuille de vaporisation (1) est soumise à une tension électrique de chauffage.

17. Procédé (30) de fabrication d'un dispositif de vaporisation (1), le dispositif de vaporisation (1) comprenant une feuille de vaporisation (2) disposée sur un élément de support (18), la feuille de vaporisation (2) formant au moins un canal d'alimentation (11) par lequel la feuille de vaporisation (2) peut être alimentée en liquide à vaporiser à partir d'un réservoir de liquide (6) via une structure de mèche (7), le canal d'alimentation (11) étant formé par un pli (14) de la feuille de vaporisation (2), le procédé (30) comprenant les étapes consistant à :
a) fournir un matériau de base (31) pour la fabrication de la feuille de vaporisation (2) comprenant un substrat (32) en polyimide avec un doublage en cuivre (33) appliqué sur une face ;
b) structurer le doublage en cuivre (33) par un procédé de gravure, de manière à former une structure en cuivre (42) sur le substrat (32) ;
c) appliquer une couche de recouvrement en polyimide (36) sur le côté du substrat (32) comportant la structure en cuivre (42) ;
d) créer une ouverture de passage (26) à l'aide d'un laser, par laquelle une face supérieure (27) de la feuille de vaporisation (2) peut être reliée fluidiquement à une face inférieure (40) de la feuille de vaporisation (2) ;
e) entamer un bord de pliage (16) à l'aide d'un laser, de manière à former une incision (37) ;
f) plier la feuille de vaporisation (2) le long du bord de pliage incisé (16) ;
g) relier la feuille de vaporisation pliée (2) à l'élément de support (18).

18. Procédé (30) selon la revendication 17, **caractérisé en ce qu'**à l'étape b) du procédé, sont exécutées les étapes partielles consistant à :
b₁) appliquer une résine photosensible (34) sur le doublage en cuivre (33) ;
b₂) exposer la résine photosensible (34) dans des zones prédéfinies ;
b₃) développer la résine photosensible (34) de manière à ce que la résine photosensible restante (34) forme une couche protectrice pour le doublage en cuivre (33) dans les zones prédéfinies ;
b₄) appliquer un agent de gravure sur le côté avec la résine photosensible développée (34) de manière à ce que le doublage en cuivre (33) ne subsiste que dans les zones prédéfinies sur le substrat (32) ;
b₃) éliminer la résine photosensible restante (34).

19. Procédé (30) selon la revendication 17 ou 18, **caractérisé en ce qu'**à l'étape c) du procédé, l'application d'une couche de recouvrement en polyimide (36) s'effectue par laminage dans une presse à vide.

20. Procédé (30) selon l'une des revendications 17 à 19, **caractérisé en ce qu'**à l'étape e) du procédé, des points de contact (38) sont en outre découverts au moyen du laser pour alimenter la feuille de vaporisation (2) en tension électrique de chauffage.

21. Procédé (30) selon l'une des revendications 17 à 20, **caractérisé en ce qu'**à l'étape e) du procédé, la feuille de vaporisation (2) est en outre découpée pour la liaison avec l'élément de support (18).
